# FASCICULE DE BREVET EUROPEEN

(11) **EP 1 974 719 B1**
(45) Date de publication et mention de la délivrance du brevet: **26.03.2014**
(21) Numéro de dépôt: 08151907.6
(22) Date de dépôt: 25.02.2008
(51) Int. Cl.: C12N 5/00, A61Q 17/04, A61Q 19/00, A61K 8/98, A61K 8/99, A61K 35/74, A61P 17/00, A61P 29/00, A61K 35/12

(54) **Milieu conditionné de culture de cellules du tractus digestif et ses utilisations**
Konditioniertes Kulturmedium von Verdauungstraktzellen, und dessen Verwendungen
Conditioned medium obtained from culture of cells from digestive tract and its uses

(30) Priorité: 26.02.2007 FR 0753496
(43) Date de publication de la demande: 01.10.2008
(73) Titulaire: L'Oréal, 75008 Paris (FR)
(72) Inventeur: Gueniche, Audrey, 92500 Rueil Malmaison (FR)
(74) Mandataire: Touroude-Barbot, Magali Linda

(56) Documents cités:
- WO-A-02/28402
- WO-A-02/098365
- WO-A-2005/077389
- WO-A-2006/037922
- SATSU HIDEO ET AL: "Food factors that regulate intestinal inflammation: Evaluation of the factors by using a coculture system" ANIMAL CELL TECHNOLOGY: BASIC AND APPLIED ASPECTS, XX, XX, 2006, pages 29-37, XP009091859
- KESHAW HUSSILA ET AL: "Bioactive glass stimulates epithelial wound healing", GASTROENTEROLOGY, vol. 130, no. 4, Suppl. 2, April 2006 (2006-04), page A487, & DIGESTIVE DISEASE WEEK MEETING/107TH ANNUAL MEETING OF THE AMERICAN-GASTROENTEROLOGICAL-ASSOCIATION; LOS ANGELES, CA, USA; MAY 19 24, 2006 ISSN: 0016-5085

## Description

La présente invention concerne des milieux de culture conditionnés et leurs utilisations, en particulier dans le domaine cosmétique ou dermatologique. Elle se rapporte ainsi au traitement et à la prévention des signes de l'irritation, de l'inflammation ou des désordres immunologiques par de tels milieux conditionnés, leurs extraits, ou les compositions les contenant. L'invention concerne également une composition comprenant l'association d'au moins un composé cosmétique ou pharmaceutique susceptible de provoquer une irritation de la peau et d'au moins un milieu de culture conditionné ou d'un des ses extraits selon l'invention.

La peau humaine est constituée de deux compartiments à savoir un compartiment superficiel, l'épiderme et un compartiment profond, le derme.
L'épiderme est composé principalement de trois types de cellules qui sont les kératinocytes (majoritaires), les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures Le derme fournit à l'épiderme un support solide. C'est également son élément nourricier. Il est principalement constitué de fibroblastes et d'une matrice extracellulaire composée elle-même principalement de collagène, d'élastine et d'une substance fondamentale. On y trouve aussi des leucocytes, des mastocytes et des macrophages tissulaires. Enfin, le derme est traversé par des vaisseaux sanguins et des fibres nerveuses.
La peau constitue une barrière contre les agressions extérieures, notamment : chimiques, mécaniques et infectieuses, et à ce titre un certain nombre de réactions de défense contre les facteurs environnementaux (climat, rayons ultraviolets, tabac, pollutions, infections ...) et/ou les xénobiotiques (comme par exemple certains médicaments) se produisent à son niveau.
Il est donc essentiel de préserver ou rétablir son intégrité et l'équilibre de ses différentes fonctions, notamment un équilibre entre les processus de renouvellement et de différenciation cellulaire, ou un degré d'hydratation optimum.

L'irritation cutanée est classiquement définie comme une réaction inflammatoire locale, réversible et non immunologique, caractérisée par un oedème et un érythème induit après un contact simple ou répété d'une substance chimique avec la peau. La dermatite irritante de contact (DIC) aiguë est principalement caractérisée par une inflammation alors que la DIC chronique est caractérisée par une hyper-prolifération des kératinocytes et une hyper-kératose transitoire. La DIC est une maladie multifactorielle dont le déclenchement dépend à la fois de facteurs intrinsèques et extrinsèques. L'âge, le fond génétique, le sexe sont autant de facteurs qui peuvent influer sur le développement de cette pathologie. De plus, les effets des irritants sont directement liés à leurs propriétés chimiques et aux concentrations appliquées qui influencent l'absorption cutanée.
L'irritation de la peau est un phénomène très important puisqu'elle représente approximativement entre 60% et 80% des cas cliniques de dermatites de contact. La majorité des autres cas représentent des dermatites allergiques de contact.
Des substances appartenant à différentes familles de produits chimiques très différents comme les solvants kératiniques, les agents déshydratants ou les agents oxydants ou réducteurs peuvent être considérés comme des irritant. A cause de cette hétérogénéité, il est très difficile de proposer une méthode pour discriminer un produit irritant en se basant sur sa structure chimique. Différents irritants peuvent induire différents types d'inflammations. En plus de leurs effets corrosifs qui induisent la libération de médiateurs de l'inflammation préformés, les produits chimiques peuvent altérer les fonctions cellulaires ou induire l'activation des cellules cutanées de l'immunité innée. En résultent la libération de nombreux composés spécifiques de l'inflammation comme des cytokines, chimiokines, compléments et des composés vasodilatateurs comme l'histamine ou les métabolites de la voie de l'acide arachidonique qui modulent l'inflammation cutanée et le recrutement cellulaire.
La pénétration cutanée des chimiques est un paramètre majeur dans l'établissement de la physiopathologie de la DIC (Norlen L et coll., J Invest Dermatol 117 :823-829, 2001, Mizutani H et coll., J Clin Invest 87 : 1066-1071, 1991). Celle-ci est liée au degré de perméabilité de la peau (qui est lié à son état physiologique) et aux propriétés physicochimiques des composés dont elle est supposée restreindre l'entrée (poids moléculaire, polarité, stade d'ionisation) et la nature de l'environnement (excipient, véhicule) par lequel ces substances sont amenées au contact de la peau.
Cette étape indispensable correspond, à partir du milieu extérieur ou du véhicule, à la libération de la molécule qui va diffuser, donc à sa mise à disposition de l'organisme.

Lors d'un contact entre un irritant et la peau, les kératinocytes sont les premières cellules à être activées par le chimique. La plupart des études sur le DIC se sont ainsi focalisées sur ce type cellulaire et de nombreuses données sont désormais connues quant à leur participation dans la physiopathologie de la DIC. Les kératinocytes jouent un rôle important dans l'initialisation de la réaction inflammatoire cutanée à travers la libération de nombreux médiateurs et de cytokines à l'origine de toute une cascade de l'inflammation aboutissant aux signes cliniques de la DIC. Parmi ceux-ci l'IL-1α et les dérivés de l'acide arachidonique revêtent une importance particulière dans le développement de l'inflammation.
La libération de l'IL-1α induit, via l'activation du facteur de transcription NF-kB, la transcription de gènes impliqués dans l'inflammation comme les cytokines IL-1β, IL-6, GM-CSF, le TNFα, les chimiokines dont l'IL-8, MCP-1, MIP-1α et l'eotaxine, ainsi que l'expression de molécules d'adhésion comme l'E-selectine ou ICAM-1 et VCAM-1 (Gordon JR, Nature 19 :346 (6281) : 274-276).

La cascade de signalisation générée à partir de l'activation des kératinocytes commence à partir de la libération de médiateurs clés pré stockés. En effet, les kératinocytes au repos contiennent de grandes quantités d'IL-1α préformée et biologiquement active (Marks F et coll., Toxicol Lett 96 :111-118, 1998), ainsi que de l'acide arachidonique (Murphy JE et coll., J Invest Dermatol 114: 602-608, 2000). Parce que ces deux composés sont constitutivement produits par les kératinocytes, et restent stockés dans la cellule, l'épiderme peut être considéré, comme un grand réservoir de médiateurs hautement inflammatoire. Une altération des keratinocytes par l'effet corrosif d'un chimique, une brûlure ou par exposition au UV, induit la libération d'IL-1α et d'acide arachidonique qui deviennent les premiers évènements de défense de l'organisme. L'IL-1α a non seulement un rôle autocrine mais a été décrite pour induire la transcription de plus de 90 gènes différents sur différents types cellulaires de peau comme le kératinocytes, les cellules endothéliales ou les fibroblastes par l'activation de la voie du facteur de transcription NF-kB. (Gordon JR, Nature 19 :346 (6281): 274-276).
L'acide arachidonique est quant à lui rapidement métabolisé en de nombreux composés hautement actifs, les eicosanoïdes comme les prostaglandines, le thromboxane et les leucotriènes agissant comme médiateurs locaux avec une faible durée de vie, impliqués dans le contrôle de la prolifération, différentiation l'apoptose ou encore la formation de l'oedème ou l'activation leucocytaire. (Murphy JE et coll., J Invest Dermatol 114 : 602-608, 2000)
Ainsi, l'IL-1α et l'acide arachidonique pourraient être considérés comme les médiateurs clés du déclenchement de l'irritation en réponse en un stress chimique (Murphy JE et coll., J Invest Dermatol 114 : 602-608, 2000).

Parmi tous les médiateurs de l'inflammation hormis les IL-1 et l'acide arachidonique, seul le TNF-α peut activer un nombre suffisant de mécanismes pour générer indépendamment une inflammation cutanée. Cette cytokine majeure de l'inflammation cutanée est déjà pré stocké dans les mastocytes dermiques (Larrick JW et coll., J Leukoc Biol 45 :429-433, 1989) mais est également produite par les kératinocytes et les cellules de Langerhans après stimulation (Groves RW, et coll., J Invest Dermatol 98 :384-387,1992). Un des mécanisme par lequel le TNF-α influence le plus la réaction inflammatoire est l'induction de molécules d'adhésion en synergie avec l'IL-1. Les molécules d'adhésion jouent un rôle essentiel dans la circulation et la pénétration des leucocytes (en particulier des neutrophiles) à partir des vaisseaux sanguins périphériques vers le derme et l'épiderme (Holliday MR et coll. Am J Contact Dermat 8 :158-164, 1997).

Une grande quantité de produits chimiques peuvent induire une irritation cutanée, cependant ils différent dans leur capacité à générer des cytokines pro-inflammatoires et une inflammation cutanée n'est pas systématiquement dépendante de la production de TNF-α.

Il est important de noter de plus, que la production d'IL-12 et d'IL-18 par les macrophages activés au site de l'inflammation joue un rôle important comme boucle d'amplification locale. En effet, ces cytokines stimulent la production d'IFN-γ par les lymphocytes T avoisinants, qui est en retour un puissant facteur de co-activation des macrophages et des kératinocytes.
De plus, une sécrétion de chimiokines, par les cellules vasculaires et les kératinocytes interviennent. Les chimiokines du sous-groupe CC (ainsi nommé car les premières cystéines sont contiguës), dont le prototype est l'IL-8, sont chimiotactiques pour les polynucléaires et certains lymphocytes. Les chimiokines du groupe CXC (ainsi nommé parce qu'un acide aminé est intercalé entre les deux premières cystéines) dont le prototype est le MCP-1 (monocyte chemoattractant protein 1), sont chimiotactiques pour les monocytes/macrophages et certains lymphocytes. Ces deux types de chimiokines libérés sur le site de l'inflammation, explique l'infiltrat cellulaire polymorphe retrouvé dans un tissu enflammé.
L'IL-6 est aussi une cytokine sécrétée par les kératinocytes, les macrophages, les cellules vasculaires activés lors d'une inflammation. Cette cytokine importante intervient dans de nombreux systèmes telles que la réponse immunitaire, l'hématopoïèse, la prolifération des ostéoclastes... Dans les irritations cutanées, l'IL-6 est produite localement et peut gagner la circulation générale et déclencher des effets régionaux et généraux.
Parallèlement aux cytokines et chimiokines ainsi qu'aux métabolites de l'acide arachidonique, un autre médiateur majeur de l'inflammation est le stress oxydatif.

Certains irritants chimiques sont connus pour générer des radicaux libres et des ROS (réactive oxygen species) capables d'induire la péroxidation des lipides ou l'altération de l'ADN. L'hypothèse que le stress oxydatif joue un rôle dans les phénomènes d'irritation induite par un chimique est supportée par le fait que des inhibiteurs/scavengers de ROS inhibe l'inflammation cutanée (Zhang L et coll., J Invest Dermatol 115 : 168-176, 2000).

Les cellules de Langerhans (LC), alors qu'elles jouent un rôle fondamental dans l'induction de réponse spécifique d'antigène ne semblent pas avoir un rôle majeur dans la physiopathologie de la DIC. De nombreuses études ont décrit des changements de morphologie ou de densité des CL après application épicutanée d'irritants (Mikulowska A et coll., Contact Dermatitis 34 :397-401, 1996 ; Kimber I et coll., J Invest Dermatol 99 : 48S-50S, 1992). Cependant ces résultats pourraient représenter plutôt une réponse non spécifique à la réaction inflammatoire générée. Il est désormais évident que l'IL-1α et le TNF-α produit lors de la DIC ont une capacité de faire migrer les LC de façon dose dépendante. Il se pourrait donc que des concentrations locales d'IL-1a et de TNF-α induite par des irritants puissent générer une migration variable de CL (Kimber I et coll., J Invest Dermatol 99 : 48S-50S, 1992).
Parmi les différentes populations du système immunitaire inné cutané, le mastocyte est une cellule majeure dans le développement de la DIC. Les mastocytes sont présents dans le derme près des vaisseaux sanguins et sont les seules cellules à contenir du TNF-α pré-stockés et biologiquement actif (Larrick JW et coll., J Leukoc Biol 45 :429-433, 1989).

A l'opposé de ces cytokines pro-inflammatoires, il existe des cytokines anti-inflammatoires tels le TGF-β et l'IL-10, ainsi que des protéines de stress connues comme modulant tout signal de « danger » (ie : BiP (grp78) et HSP27) (Panayi G et coll, Cur Opinion Immunol 16 : 531-534, 2004).

Il est donc souhaitable de trouver de nouveaux moyens de renforcer la résistance de la peau à ces mécanismes délétères, et donc d'éviter, traiter ou prévenir l'inflammation et les désordres immunologiques.

La demande WO 02/098365 (Advanced Tissue Science) propose l'utilisation de milieux de culture conditionnés pour des applications cosmétiques ou pharmaceutiques. Les milieux conditionnés sont obtenus par culture de cellules de la peau humaine, en particulier de fibroblastes ou kératinocytes. Ces cellules sont génétiquement modifiées pour augmenter leur production de facteurs de croissance ou d'antioxydants dans le milieu, qui contient généralement un collagène soluble.

US 2005/0249691 décrit des compositions cosmétiques ou dermatologiques comprenant un milieu de culture pour des cellules de la peau ou des couches cornées, en association avec une matrice gélifiée ; les compositions contiennent obligatoirement du collagène, des chitosanes et des glycosaminoglycanes.

US 2006/0182701 décrit également des compositions cosmétiques ou dermatologiques comprenant un milieu de culture de cellules de la peau. Il vise à fournir aux cellules de la peau sur lesquelles elles sont appliquées un milieu qui permettra leur développement analogue à ce qui est obtenu in vitro.

De manière inattendue, il a maintenant été trouvé dans le cadre de la présente invention qu'un milieu de-culture conditionné par des cellules complètement différentes des cellules cutanées, peut être utilisé pour favoriser le bon état de la peau ou de ses annexes et lutter contre l'inflammation et/ou l'irritation et/ou les désordres immunologiques.

C'est pourquoi la présente invention a pour objet l'utilisation d'au moins un milieu de culture cellulaire conditionné ou d'un extrait de celui-ci, pour la préparation d'une composition destinée au traitement des signes de l'inflammation et/ou des désordres immunitaires, ledit milieu étant susceptible d'être obtenu par contact avec au moins une culture de cellules du tractus digestif, au moins des microorganismes probiotiques et en outre des cellules du sang périphérique.

Le milieu de culture cellulaire, aussi appelé milieu conditionné selon la présente invention, est susceptible d'être obtenu par contact avec des cellules du sang périphérique, ou des cellules dérivés de cellules de sang périphérique. De telles-cellules sont en particulier des leucocytes, mais pourront également être choisies parmi des lignées cellulaires immortalisées, dérivées de lignées de cellules sanguines, notamment de monocytes; de manière non limitative on peut citer des cellules commercialisées par la société LGC Promochem, sous les désignations suivantes:
SC (code ATCC : CRL-9855)
AML-193 (code ATCC : CRL-9589)
THP-1 (code ATCC : TIB-202).

Ces cellules de sang périphériques ou leurs dérivées, notamment les leucocytes peuvent par exemple être en culture dans le milieu avec les cellules de tractus digestif.

De préférence, ces leucocytes comprennent majoritairement des lymphocytes, mais peuvent aussi comporter d'autres cellules de sang périphérique telles que les monocytes, qui sont mises en contact avec le milieu.

Selon l'un des modes de réalisation avantageux de l'invention, la culture de cellules du tractus digestif est une culture tridimensionnelle.

Les cellules du tractus digestif utiles pour la mise en oeuvre de l'invention pourront être dérivées de différentes parties du tractus digestif, tel que l'oesophage, l'estomac ou l'intestin. Avantageusement on utilise des cellules dérivées de l'épithélium intestinal; de telles cellules épithéliales de l'intestin sont connues de l'homme du métier. On peut citer, à titre d'exemple non limitatif, les cellules humaines d'origine intestinale connues sous l'appellation CaCO-2, HT29 ou T84.
Les souches correspondantes sont déposées dans les collections de culture de l'ATCC avec les n° suivants:
Caco 2: ATCC N° HTB-37
HT29: ATCC N° HTB-38
T84: ATCC N° CCL-248

Au sens de la présente invention, on entend par "microorganisme probiotique", un microorganisme vivant qui, lorsqu'il est consommé en quantité adéquate, a un effet positif sur la santé de son hôte "joint FAO/WHO Expert Consultation on Evaluation of Health and Nutritional Properties of Probiotic in Food Including Powder Milk with Live Lactic Acid Bacteria, 6 octobre 2001", et qui peut en particulier améliorer l'équilibre microbien intestinal.
Les probiotiques peuvent être introduits dans le milieu de culture cellulaire sous forme d'une suspension de cellules vivantes, dont la concentration sera adaptée par l'homme du métier en fonction de la quantité des autres constituants du mélange. A titre indicatif, on peut utiliser un inoculum comprenant environ 10⁴ à 10⁹ufc/ml (ufc signifiant "unité formant une colonie ou cfu signifiant "colony forming unit" c'est-à-dire "unité capable de former une colonie"), de préférence au moins 10⁵ cfu/ml. L'inoculum de probiotiques peut de façon classique être à une concentration d'environ 10⁶ à 10⁷ cfu.

Les microorganismes convenant à l'invention peuvent être choisis notamment parmi les ascomycetes telles que *Saccharomyces, Yarrowia, Kluyveromyces, Torulaspora, Schizosaccharomyces pombe, Debaromyces, Candida, Pichia, Aspergillus* et *Penicillium,* des bactéries du genre *Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus, Lactobacillus* et leurs mélanges.
Comme ascomycetes convenant tout particulièrement à la présente invention, on peut en particulier citer *Yarrowia lipolitica et Kluyveromyces lactis,* de même que *Torulaspora, Schizosaccharamyces pombe, Candida* et *Pichia* ou encore des levures comme *Saccharomyces cerevisiae* ou *boulardii.*
En ce qui concerne les microorganismes probiotiques, ce sont les genres bactériens et de levure suivants qui sont généralement utilisés :
- les bactéries lactiques : qui produisent par fermentation du sucre de l'acide lactique. Suivant leur morphologies ont les divisent en deux groupes :
   - *Lacobacillus species : Lactobacillus acidophilus (LC1, NCFB 1748) ; amylovorus, casei (Shirota), rhamnosus* (souche GG), *brevis, crispatus, de*/*brueckii (subsp bulgaricus, lactis), fermentum, helveticus, gallinarum, gasseri johnsonii, paracasei, plantarum, reuteri, rhamnosus, salivarius), alimentarius, curvatus, casei subsp. casei, sake*
   - *Gocci : Enterococcus (faecalis, faecium), Lactococcus lactis (subspp lactis ou cremoris), Leuconstoc mesenteroides* subsp *dextranicum, Pediococcus acidilactici, Sporolactobacillus inulinus, Streptococcus salvarius subsp. Thermophilus, Streptococcus thermophilus, Staphylococccus carnosus, Staphylococcus xylosus*
- Les bifidobactéries ou *Bifidobacterium species : Bifidobacterium adolescentis, animalis, bifidum, breve, lactis, longum, infantis, pseudocatenulatum*
- Les autres bactéries sporulées : *Bacillus (cereus var toyo ou subtilis), Bacillus coagulans, Bacillus licheniformis, Escherichia coli strain nissle, Propionibacterium freudenreichii,*
Des exemples spécifiques de microorganismes probiotiques sont *Bifidobacterium adolescentis, Bifidobacterium animalis, Bifidobacterium bifidum, Bifidobacterium breve, Bifidobacterium lactis, Bifidobacterium longum, Bifidobacterium infantis, Bifidobacterium pseudocatenulatum, Lactobacillus acidophilus (LC1, NCFB 1748) ; Lactobacillus amylovorus, Lactobacillus casei (Shirota), Lactobacillus rhamnosus (*souche GG*)*, *Lactobacillus brevis, Lactobacillus crispatus, Lactobacillus delbrueckii (subsp bulgaricus, lactis), Lactobacillus fermentum, Lactobacillus helveticus, Lactobacillus gallinarum, Lactobacillus gasseri, Lactobacillus johnsonii, Lactobacillus paracasei, Lactobacillus plantarum, Lactobacillus reuteri, Lactobacillus rhamnosus, Lactobacillus salivarius), Lactobacillus alimentarius, Lactobacillus curvatus, Lactobacillus casei subsp. casei, Lactobacillus* sake *Lactococcus lactis, Enterococcus (faecalis, faecium), Lactococcus lactis (subspp lactis ou cremoris), Leuconstoc mesenteroides* subsp *dextranicum, Pediococcus acidilactici, Sporolactobacillus inulinus, Streptococcus salvarius subsp. Thermophilus, Streptococcus thermophilus, Staphylococccus carnosus, Staphylococcus xylosus, Saccharomyces (cerevisiae* ou encore *boulardii), Bacillus (cereus var toyo ou subtilis), Bacillus coagulans, Bacillus licheniformis, Escherichia coli strain nissle, Propionibacterium freudenreichü* et leurs mélanges.
Avantageusement, au moins un microorganisme probiotique est choisi parmi les bactéries lactiques, les bifidobactéries et les levures Saccharomyces.
Plus particulièrement, il s'agit de microorganismes probiotiques issus du groupe des bactéries lactiques, comme notamment les *Lactobacillus* et/ou les *Bifidobacterium,* en particulier les *Lactobacillus.* A titre illustratif de ces bactéries lactiques, on peut plus particulièrement citer les *Lactobacillus johnsonii, Lactobacillus reuteri, Lactobacillus rhamnosus, Lactobacillus paracasei, Lactobacillus casei* ou *Bifidobacterium bifidum, Bifidobacterium breve, Bifidobacterium longum, Bifidobacterium animalis, Bifidobacterium lactis, Bifidobacterium infantis, Bifidobacterium adolescentis* ou *Bifidobacterium pseudocatenulatum* et leurs mélanges.
Les espèces convenant tout particulièrement sont les *Lactobacillus johnsonii, Lactobacillus paracasei, Bifidobacterium adolescentis, Bifidobacterium longum* et *Bifidobacterum Lactis NCC 2818 (encore désigné Bb12 ATCC 27536) ;* on peut citer en particulier les souches suivantes, déposées suivant le traité de Budapest à la collection de culture de l'Institut Pasteur (28 rue du Docteur Roux, F-75024 Paris cedex 15) les 30/06/92, 12/01/99, 15/04/99, 15/04/99, 07/06/05: *Lactobacillus johnsonii* (CNCM I-1225), *Lactobacillus paracasei* (CNCM I-2116), *Bifidobacterium adolescentis* (CNCM I-2168) et *Bifidobacterium longum* (CNCM I-2170) et *Bifidobacterum Lactis* (CNCM I-3446), et le genre *Bifidobacterium longum (BB536).* La souche de *Bifidobacterium lactis* CNCM I-3446 peut être obtenue chez Hansen (Chr. Hansen A/S, 10-12 Boege Alle, P.O. Box 407, DK-2970 Hoersholm, Danemark).

Le milieu de culture cellulaire avec lequel les cellules sont mises en contact sera tout milieu nutritif convenant à la survie et/ou la culture des différents types cellulaires mis en oeuvre. Il contient généralement une source de carbone et d'azote, des minéraux, des vitamines et/ou des oligoélements, comme par exemple des acides aminés, des sucres, des protéines, des acides gras.
Les cellules peuvent être cultivées en dehors de leur tissu d'origine. Pour cela elles doivent être cultivés dans un environnement proches de leur condition naturelle dans le tissu. Ces cultures requièrent des facteurs vitaux dans leur milieu de culture. Cet environnement doit avoir une composition définie composée de minéraux et de biomatériaux connu sous le nom de milieu de culture. Ces milieux de culture sont en général fournis par des fournisseurs spécialistes et possèdent des caractéristiques particulières en fonction des types cellulaires. Les milieux de culture contiennent généralement en plus de l'eau, une source de carbone et d'azote, des phosphates et des sulfates, des minéraux et des facteurs de croissances et des vitamines en quantité adéquat.
Les cellules cultivées dans ce type de milieu nécessitent souvent l'ajout de sérum. Ce sérum a une composition complexe et apporte aux cellules au moins des hormones, des facteurs d'adhésion et des acides aminés. Ce sérum pourra par exemple être remplacé, en tout ou partie, par l'ajout des milieux conditionnés de l'invention. En effet, les milieux conditionnés pourront aussi être ajoutés en plus dans le milieu de culture comme enrichissement.

L'homme du métier déterminera aisément des milieux adaptés. On peut citer, de manière non limitative, le milieu RPMI 1640, le Dulbecco's Modified Eagle's Medium (DMEM), le Minimal Essential Medium (MEM), le M199, le RPMI 1640 ou l'Iscove's Modified Dulbecco's Medium (EDMEM), le Ham's F-12, le Ham's F-10, le NCTC 109 et le NCTC 135.
Ces milieux peuvent être complémentés par tout additif classiquement utilisé en culture cellulaire tel que, par exemple et de manière non limitative, des précurseurs de phospholipides, des acides aminés non essentiels, des acides nucléiques, des vitamines, des antibiotiques, des co-facteurs enzymatiques, des sels minéraux, de l'insuline, de la transferrine, de la triiodothyronine, de l'éthanolamine, de l'o-phosphoryl-éthanolamine ou des facteurs de croissance tels que le facteur de croissance nerveuse ou la neurotrophine-3.
Les concentrations des différents additifs usuellement utilisés pour complémenter les milieux de culture cellulaire peuvent être déterminer et adapter par l'homme de l'art, notamment selon le type de cellules à cultiver.
D'autres milieux sont décrits dans HAM and McKEEHAN, « Methods in Enzymology », 58:44-93, 1979, ou encore dans BOTTENSTEIN et al., « Methods in Enzymology », 58:94-109, 1979, dont le contenu est incorporé ici par référence.
Par ailleurs, il est également possible d'utiliser des mélanges de différents milieux notamment des milieux précités, tels que par exemple un mélange de DMEM/HAM F12.

Ces milieux peuvent être supplémentés en facteurs de croissance spécifique, ou par exemple avec du sérum, mais ce dernier composant peut aussi être absent.
Selon un mode de réalisation, on n'ajoute pas de collagène dans le milieu de culture.
Ce milieu de culture peut être liquide, semi-liquide, gélifié ou solide, de préférence au moins partiellement liquide.

Par extrait du milieu de culture cellulaire conditionné on entend notamment toute fraction ou sous composé de ces milieux conditionnés obtenus par dialyse, fractionnement, séparation de phase, chromatographie par filtration, chromatographie par affinité, précipitation, concentration, lyophilisation....
Les milieux conditionnés peuvent être générés à partir de milieux qui peuvent être dépourvus de sérum et de produit animal. Ce milieu conditionné est de préférence obtenu après stimulation des cellules du tractus digestif en présence de leucocytes humains, par des probiotiques et en particulier des probiotiques de l'espèce des lactobacilles ou des bifidobactéries.

Avantageusement, le milieu de culture (ou ses extraits) utilisé dans les compositions de l'invention est un milieu stabilisé, c'est-à-dire qu'il a subi une manipulation destinée à le préserver dans l'état dans lequel il se trouvait à un instant donné choisi, généralement à la fin de son processus de préparation, tout en ayant conservé ses propriétés intrinsèques. En particulier, cette manipulation est destinée à rendre ledit milieu stérile, c'est-à-dire incapable de permettre la croissance de microorganismes tout en préservant les propriétés biologiques dont il est doté. La stabilisation du milieu de culture peut être obtenue par toute technique connue de l'homme du métier, comme par exemple la filtration stérilisante, l'autoclavage, l'ultra haute température (technique UHT), la stérilisation haute pression, les rayonnements γ ou la congélation.
Les milieux de culture conditionnés selon l'invention contiennent généralement de l'IL-10, qui n'était pas présent dans les différents constituants du milieu d'origine. Les quantités d'IL-10 pourront varier selon les conditions de préparation du milieu conditionné de l'invention, mais correspondront en général à une concentration supérieure ou égale à 20 pg/ml de milieu récupéré après contact avec les différents types cellulaires.

Le milieu de culture conditionné selon l'invention, ses extraits ou les compositions le contenant sont particulièrement utiles pour prévenir, diminuer ou traiter les signes de l'irritation cutanée.

Les réactions cutanées, notamment l'irritation cutanée, peuvent être induites par un stress exogène, d'origine chimique, par exemple, xénobiotiques, antigènes, allergènes, produits chimiques, composés susceptibles de provoquer une irritation de la peau, peeling, d'origine environnementale (température, climat, rayonnement UV, pollution atmosphérique, notamment métaux lourds, ozone, fumée de cigarette...) ou encore d'origine mécanique (frottements, rasage) et tout stress d'origine endogène tels que les désordres impliquant un mécanisme inflammatoire et/ou hormonal affectant la peau, une muqueuse, le cuir chevelu et/ou les cheveux.
Le stress endogène physiologique peut par exemple être lié à la production anormale de médiateurs proinflammatoires (neuromediateurs, cytokine, chemokines) ou à une alopécie androgénétique.

L'invention a de plus pour objet ladite utilisation selon la présente invention, caractérisée en ce que ledit milieu conditionné ou ses extraits est destiné à prévenir et/ou diminuer ladite réaction cutanée induite par au moins une condition choisie parmi l'action de xénobiotiques, d'antigènes, d'allergènes, de produits chimiques, de composés susceptibles de provoquer une irritation de la peau, d'un peeling, l'action de la température, du climat, de rayonnements UV, de la pollution atmosphérique, ou encore par les frottements, la production anormale de médiateurs proinflammatoires et l'alopécie androgénétique.

Le milieu conditionné ou ses extraits peut en particulier être destiné à prévenir et/ou diminuer l'effet irritant d'une composition cosmétique ou dermatologique contenant un ou plusieurs composés susceptibles de provoquer une irritation.

L'invention a encore pour objet l'utilisation d'au moins un milieu conditionné ou ses extraits pour la préparation d'une composition destinée à prévenir et/ou traiter les désordres cutanés liés à une irritation cutanée, par exemple rencontrée chez les sujets présentant des peaux et/ou muqueuses et/ou cuirs chevelus irritables et/ou allergiques.

L'invention a plus particulièrement pour objet ladite utilisation pour la préparation d'une composition destinée à prévenir et/ou traiter l'irritation cutanée, caractérisée en ce que l'irritation cutanée est induite par au moins une condition choisie parmi l'action de xénobiotiques, d'antigènes, d'allergènes, de produits chimiques, de composés susceptibles de provoquer une irritation de la peau, d'un peeling, l'action de la température, du climat, de rayonnements UV, de la pollution atmosphérique, ou encore par les frottements, la production anormale de médiateurs proinflammatoires et l'alopécie androgénétique.

L'invention a également pour objet l'utilisation d'au moins un milieu conditionné ou ses extraits pour la préparation d'une composition destinée à prévenir et/ou traiter des désordres cutanés liés à une réaction cutanée, par exemple de type inflammatoire ou immuno-allergique.

Ainsi, les milieux conditionnés ou les compositions selon l'invention sont plus particulièrement utiles pour lutter contre les désordres cutanés choisis parmi les dartres, les oedèmes et/ou boutons, l'érythème inflammatoire ; les prurits ; le psoriasis, l'atopie cutanée, la dermatite atopique ; l'urticaire; les dermites de contact, l'eczéma ; les dermatites séborrhéiques ; l'acné ; les hyperpigmentations inflammatoires ; les dermatoses immunes ; les maladies immunes bulleuses ; la sclérodermie ; l'élastose actinique ; les pelades ou *alopecia areata* ; le vitiligo ; le lupus érythémateux systémique ; le *pemphigus vulgaris* ; les épidermolyses bulbeuses dystrophiques et la canitie d'origine autoimmune.

En effet, on sait que certains désordres cutanés sont liés à une réaction cutanée de type inflammatoire ou immuno-allergique, parmi lesquels l'érythème inflammatoire, le psoriasis, l'atopie cutanée, la dermatite atopique, les réactions allergiques de type hypersensibilité immédiate, telles que l'urticaire, les réactions allergiques de type hypersensibilité retardée, telles que les dermites de contact, l'eczéma ; les dermatites séborrhéiques, l'acné, les hyperpigmentations inflammatoires, les dermatoses immunes, l'élastose actinique, les pelades ou *alopecia areata* ; le vitiligo ; le lupus érythémateux systémique ; *le pemphigus vulgaris* ; les épidermolyses bulbeuses dystrophiques et la canitie d'origine autoimmune.
De plus, au niveau de la peau, les expositions UV génèrent des réactions strictement inflammatoires et ont des effets irritants, qui peuvent entraîner le développement d'un érythème, d'un oedème et/ou d'une hyperkératose. Ces réactions inflammatoires et/ou d'irritation sont liées à une stimulation d'acteurs spécifiques du système immunitaire.

Selon l'un des modes de réalisation de l'invention, le milieu conditionné ou les compositions selon l'invention sont destinés à protéger les cellules cutanées des dégâts causés par les rayonnements UV.
Le milieu conditionné ou les compositions selon l'invention sont avantageusement destinés à protéger les cellules, en particulier les cellules cutanées des dégâts de leur ADN.

Les compositions sont notamment utiles pour :
1) réparer l'ADN altéré et ainsi réduire le risque de cancer (par exemple induit au niveau cutané à la suite de stress lié à l'environnement). Ces substances pourraient notamment influencer le principal mécanisme de réparation du matériel génétique chez les mammifères, nucléoside-excision repair
2) inhiber les caspases dans les cas de dermatites atopiques ou d'allergie de contact inhiber les cellules T activées infiltrant la peau des sujets atteints qui induisent l'apoptose des kératinocytes avoisinant. Et ainsi, pourrait réduire le nombre de kératinocytes apoptotiques, et donc la perte de la cohésion intercellulaire (acantholyse) et en conséquence la formation de vésicule.

Ainsi, on utilise des compositions cosmétiques contenant par exemple des actifs kératolytiques et/ou desquamants, pour lutter contre le vieillissement, et notamment des actifs exfoliants et /ou des actifs favorisant le renouvellement cellulaire, tels que les α -hydroxy-acides (notamment acides lactique, glycolique, citrique), les β -hydroxy-acides (notamment acides salicylique, n-octanoyl-5-salicylique) et les rétinoïdes (notamment acide rétinoïque tout trans ou 13-cis, rétinol). Malheureusement, si ces actifs sont utilisés en des quantités trop importantes, ils peuvent provoquer une irritation cutanée. L'utilisation de ces composés notamment pour les utilisateurs à peaux et/ou cuirs chevelus irritables et/ou allergiques doit donc être limitée.

En outre, même certains composés qui sont considérés comme inertes dans une composition cosmétique ou dermatologique, tels que, par exemple, les conservateurs, les tensioactifs, les parfums, les solvants ou les propulseurs, peuvent présenter un caractère irritant lorsqu'ils sont appliqués sur les matières kératiniques et notamment la peau y compris le cuir chevelu chez des sujets à peau irritable et/ou allergique, ce caractère irritant dépendant du composé utilisé et de la sensibilité de la peau et de la flore cutanée résidente de l'utilisateur.

Les composés susceptibles de provoquer une irritation de la peau sont généralement utilisés en des doses faibles. L'utilisation à faible quantité de ces composés peut alors s'avérer peu avantageuse par rapport à l'utilisation d'autres composés moins actifs, mais moins ou pas irritants et donc utilisés en plus grande quantité, ou par rapport à la finalité du composé, telle que par exemple, la stabilité de la composition quand il s'agit d'émulsionnants ou la bonne conservation de la composition quand il s'agit de conservateurs.

Il existe donc un besoin de trouver des composés à effet apaisant capables notamment de prévenir et/ou diminuer l'effet irritant de compositions cosmétiques ou dermatologiques contenant un ou plusieurs composés susceptibles de provoquer une irritation de la peau, et de prévenir et/ou traiter des désordres cutanés liés à une irritation cutanée de type inflammatoire ou immuno-allergique.

La demande décrit également l'utilisation d'au moins un milieu conditionné ou ses extraits pour la préparation d'une composition, caractérisée en ce que ledit milieu conditionné ou ses extraits est destiné à prévenir et/ou diminuer l'effet irritant d'une composition cosmétique ou dermatologique contenant un ou plusieurs composés susceptibles de provoquer une irritation de la peau.

L'utilisation selon la présente invention est particulièrement destinée au traitement des peaux et/ou muqueuses et/ou cuirs chevelus irritables et/ou allergiques.

L'invention a en outre pour objet une composition pour application topique sur la peau et/ou les muqueuses et/ou le cuir chevelu comprenant, dans un milieu physiologiquement acceptable :
- au moins un composé cosmétique ou pharmaceutique susceptible de provoquer une irritation de la peau et,
- au moins un milieu conditionné ou ses extraits.

L'utilisation d'au moins un milieu conditionné ou ses extraits présente donc entre autre l'avantage de supprimer l'irritation cutanée qu'auraient pu provoquer les composés à effet secondaire irritant, et aussi de permettre d'augmenter la quantité desdits composés dans des compositions cosmétiques ou dermatologiques par rapport à la quantité normalement utilisée, en vue d'une efficacité accrue de ces derniers.

Ainsi, on peut utiliser, y compris dans les compositions destinées aux peaux et/ou aux muqueuses et/ou aux cuirs chevelus irritables et/ou allergiques, des agents susceptibles de provoquer une irritation de la peau tels que des actifs cosmétiques (ex : agent kératolytiques et/ou desquamant), des actifs dermatologiques (ex : rétinoïdes), certains tensioactifs, conservateurs, parfums, solvants, propulseurs, et leurs mélanges pour autant que lesdites compositions comprennent au moins un milieu conditionné ou ses extraits.

Par "peaux et/ou muqueuses et/ou cuirs chevelus irritables et/ou allergiques" selon l'invention, on entend notamment des peaux et/ou muqueuses et/ou cuirs chevelus qui réagissent aux agressions extérieures, parfois de façon exagérée. Ces peaux et/ou muqueuses et/ou cuirs chevelus sont dès lors davantage sujets au développement d'une réaction cutanée pouvant se manifester par des rougeurs, des prurits et/ou impliquer des mécanismes immunologiques ou inflammatoires, par opposition aux peaux sensibles.

Dans la présente description, à moins qu'il n'en soit spécifié différemment, on entend par peau l'ensemble du revêtement du corps humain, c'est-à-dire la peau, les muqueuses et le cuir chevelu.
Par phanères on entend les ongles, les cheveux et les poils, tels que les cils.

La quantité de milieu de culture conditionné ou de ses extraits selon l'invention dans les compositions sera adapté par l'homme du métier pour obtenir l'effet recherché. La quantité efficace sera ainsi déterminée par des techniques de routine, comprenant des essais in vitro et des dosages in vivo, et dépendra notamment du type d'extrait utilisé et du type de formulation retenue.
A titre indicatif, la concentration de matière active de milieu conditionné pourra être comprise entre 0,001 et 50 % en poids par rapport au poids total de la composition, notamment inférieure ou égale à 10%, mais ces quantités pourront varier sans inconvénient.

Les compositions selon l'invention contiennent également un milieu physiologiquement acceptable, en particulier un milieu cosmétiquement ou pharmaceutiquement acceptable. Il s'agit plus particulièrement de compositions cosmétiques ou pharmaceutiques, notamment de compositions dermatologiques.

Par composition ou produit cosmétique au sens de l'invention on entend notamment toute substance ou préparation destinée à être mise en contact avec les diverses parties superficielles du corps humain (épiderme, système pileux et capillaire, ongles, lèvres et organes génitaux externes) ou avec les dents et les muqueuses buccales en vue, exclusivement ou principalement, de les nettoyer, de les parfumer, d'en modifier l'aspect et/ou de corriger les odeurs corporelles et/ou de les protéger ou de les maintenir en bon état (directive cosmétique 76/768/CEE modifiée).
Ces compositions ont généralement une odeur et un aspect rendant agréable leur application sur le corps humain.

De préférence, une composition de l'invention est appliquée sur la peau ou les muqueuses.
Selon le mode d'administration considéré, elle peut se présenter sous toutes les formes galéniques normalement utilisées.
Pour une application topique sur la peau ou les muqueuses, la composition peut avoir la forme notamment de solutions aqueuses ou huileuses ou de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique ou de mousses. Elles peuvent également se présenter sous forme de microsphères ou nanosphères ou de vésicules lipidiques ou polymériques ou de patchs polymériques et d'hydrogels permettant une libération contrôlée.
Selon un mode de réalisation avantageux, la composition est une composition dermocosmétique contenant, dans un support cosmétiquement ou pharmaceutiquement acceptable, au moins un un milieu conditionné ou un extrait selon l'invention à raison d'au moins 0,001% en poids par rapport au poids total de la composition, et de préférence de 0,05 à 3%.
Ces compositions sont préparées selon les méthodes usuelles.

Les quantités des différents constituants des compositions selon l'invention sont celles classiquement utilisées dans les domaines considérés. Les constituants et leurs quantités seront de préférence choisis de manière à ne pas interagir, en la diminuant, avec l'activité du milieu conditionné ou de ses extraits.
Dans le domaine de la cosmétique, ces compositions constituent notamment des crèmes de nettoyage, de protection, de traitement ou de soin pour le visage, pour les mains, pour les pieds, pour les grands plis anatomiques ou pour le corps (par exemple crèmes de jour, crèmes de nuit, crèmes de démaquillage, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions de bronzage artificiel, des compositions pour le bain, des compositions désodorisantes comprenant un agent bactéricide, des gels ou lotions après-rasage.
Les compositions selon l'invention peuvent également consister en des préparations solides constituant des savons ou des pains de nettoyage.
Les compositions peuvent aussi être conditionnées sous forme de composition pour aérosol comprenant également un agent propulseur sous pression.
Une composition selon l'invention peut aussi être une composition pour les soins du cuir chevelu, et notamment un shampoing, une lotion de mise en plis, une lotion traitante, une crème ou un gel coiffant, de lotions restructurantes pour les cheveux, une lotion ou un gel antichute, un shampoing antiparasitaire, antipelliculaire etc.
Une composition peut aussi être à usage bucco-dentaire, par exemple une pâte dentifrice. Dans ce cas, la composition peut contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut varier d'environ 5 % à 80 % en poids, et de préférence d'environ 5 % à 50 % en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3 % à 30 % en poids, et de préférence de 0,5 % à 20 % en poids par rapport au poids total de la composition. L'émulsion peut, en outre, contenir des vésicules lipidiques.
Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeurs et les matières colorantes. Les quantités de ces différents adjuvants sont celles classiquement utilisées dans le domaine cosmétique, et par exemple varient d'environ 0,01 % à 10 % du poids total de la composition. Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique). Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{®} 63 par la société Gattefosse.

Comme solvants utilisables dans l'invention, on peut citer les alcools inférieurs notamment l'éthanol et l'isopropanol et le propylène glycol.
Comme gélifiants hydrophiles utilisables dans l'invention, on peut citer les polymères carboxyvinyliques (carbomer^{®}), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, l'éthylcellulose et le polyéthylène.

### INGREDIENTS COSMETIQUE OU PHARMACEUTIQUE SUSCEPTIBLE DE PROVOQUER UNE IRRITATION DE LA PEAU

L'utilisation non thérapeutique vise notamment à prévenir et/ou diminuer l'effet irritant d'une composition cosmétique ou dermatologique contenant ou plusieurs composés susceptibles de provoquer une irritation de la peau.

Une composition selon la présente invention comprend au moins un composé cosmétique ou pharmaceutique susceptible de provoquer une irritation de la peau.

Parmi ces composés on peut notamment citer des composés ou actifs cosmétiques, des composés ou actifs dermatologiques, des tensioactifs notamment des tensioactifs anioniques, des conservateurs, des détergents, des parfums et notamment des solutions alcooliques parfumantes, des solvants, des propulseurs et leurs mélanges.

Plus particulièrement à titre d'actifs dermatologiques ou cosmétiques on peut citer certains agents desquamants qui peuvent être également des agents de peeling.

Parmi les agents spécifiquement de peeling on peut citer des particules abrasives/exfoliantes de sources minérales, organiques, naturelles ou synthétiques. On peut plus particulièrement citer les particules de pierre ponce, de silice, des billes de polyéthylènes, de nylon et des poudres de noyaux de fruits.

Parmi ces agents desquamants les suivants sont susceptibles de provoquer une irritation de la peau : les acides monocarboxyliques saturés (acide acétique) et insaturés, les acides dicarboxyliques saturés et insaturés, les acides tricarboxyliques saturés et insaturés ; les α-hydroxyacides et β-hydroxyacides des acides monocarboxyliques ; les α-hydroxyacides et β-hydroxyacides des acides dicarboxyliques ; les α-hydroxyacides et β-hydroxyacides, des acides tricarboxiliques, les cétoacides, les α-cétoacides, les β-cétoacides d'acides polycarboxyliques, d'acides polyhydroxy monocarboxyliques, d'acides polyhydroxy bicarboxyliques et d'acides polyhydroxy tricarboxyliques.

Particulièrement parmi les α-hydroxyacides ou leurs esters on peut citer : les acides glycolique, dioïque comme l'acide octadécène dioïque ou Arlatone dioc DCA vendu par la société Uniqema, citrique, lactique, tartrique, malique ou mandélique, leur esters comme le tartrate de dialkyle (C12/C13) ou Cosmacol ETI, le citrate de tri-alcools C12-13 ramifiés ou Cosmacol ECI commercialisé par la société SASOL.

Parmi les β-hydroxyacides on peut citer : l'acide salicylique et ses dérivés (dont l'acide n-octanoyl 5-salicylique).

Parmi les α-cétoacides on peut citer l'acide ascorbique et ses dérivés.

Parmi les autres agents desquamants on peut citer : les acides pyruvique, gluconique, glucuronique, oxalique, malonique, succinique, acétique, gentisique, cinnamique, azélaique ; le phénol ; la résorcine ; l'urée et ses dérivés, l'hydroxyéthyl urée ou hydrovance® de chez NATIONAL STARCH ; les oligofucoses ; l'acide jasmonique et ses dérivés ; l'acide ascorbique et ses dérivés, l'acide trichloracétique ; l'extrait de Saphora japonica et le résvératrol.

Parmi les agents desquamants, ceux capables d'agir sur les enzymes impliqués dans la desquamation ou la dégradation des cornéodesmosomes peuvent également être susceptibles de provoquer une irritation de la peau.

Parmi ceux-ci, on peut notamment citer les agents chélatants des sels minéraux tels l'EDTA ; l'acide N-acyl-N,N',N'éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP 0 852 949, ainsi que le méthyl glycine diacétate de sodium commercialisé par BASF sous la dénomination commerciale TRILON M^{®}); le miel ; les dérivés de sucre tels que l'O-octanoyl-6-D-maltose, le O-linoleyl-6-D-glucose et la N-acétyl glucosamine.

Les rétinoïdes sont également des composés susceptibles de provoquer une irritation de la peau. On peut par exemple citer parmi eux le rétinol et ses esters, le rétinal, l'acide rétinoïque et ses dérivés tels que ceux décrits dans les documents FR-A-2 570 377, EP-A-199 636, EP-A-325 540, EP-A-402 072, et l'adapalène.

Les sels et dérivés, comme les formes cis ou trans, les mélanges racémiques, les formes dextrogyres ou levogyres des composés cités précédemment sont aussi considérés comme des composés susceptibles de provoquer une irritation de la peau.

D'autres actifs dermatologiques ou cosmétiques susceptibles de provoquer une irritation de la peau sont également cités ci-après :
- l'urée et ses dérivés comme l'hydroxyéthyl urée ou hydrovance® de NATIONAL STARCH,
- certaines vitamines telles que la vitamine D et ses dérivés tels que la vitamine D3, la vitamine D2, le calcitriol, le calcipotriol, le tacalcitol, la 24,25-diOH vitamine D3, la 1-OH vitamine D2 et la 1,24-diOH vitamine D2 ; la vitamine B9 et ses dérivés,
- les peroxydes comme le peroxyde de benzoyle, l'eau oxygénée,
- les antichutes tels que le minoxidil et ses derivés tel que l'aminexil,
- les teintures et les colorants capillaires comme les aminophénols et leurs dérivés tels que la para-phénylène diamine (p-PDA), la N-phenyl p-PDA, le toluène 2,5-diamine sulfate, la méta-phénylène diamine (m-PDA), la toluène 3,4-diamine et l'ortho-phénylène diamine (o-PDA),
- les agents anti-transpirants comme les sels d'Aluminium, tel que l'hydroxychlorure d'aluminium,
- les déodorants,
- les actifs dépilatoires et/ou de permanentes tels que les thioglycolates, l'ammoniaque,
- le thioglycolate et ses sels,
- le phénoxyéthanol,
- le 1,2-pentanediol,
- les solutions alcooliques parfumantes (parfums, eaux de toilette, après rasage, déodorants)
- les anthralines (dioxyanthranol),
- les anthranoïdes (par exemple ceux décrits dans le document EP-A-319028),
- les sels de lithium,
- les dépigmentants (ex : hydroquinone, vitamine C à forte concentration, acide kojique),
- certains actifs amincissants à effet chauffant,
- les nicotinates et leurs dérivés,
- la capsaïcine,
- les actifs antipoux (pyréthrine),
- les antiprolifératifs tels que le 5-fluoro uracile ou le méthotrexate,
- les agents antiviraux,
- les antiparasitaires,
- les antifongiques,
- les antiprurigineux,
- les antiséborrhéiques,
- les propigmentants tels que les psoralènes et les méthylangécilines, et
- leurs mélanges.

Comme conservateurs, on peut citer le phénoxyéthanol, la chorehexidine et le chlorure de benzalkonium.

Comme tensioactifs, on peut citer les tensioactifs anioniques, cationiques et amphotères, plus particulièrement les tensioactifs anioniques tels que les alkyl sulfates et alkyl éther sulfates comme le lauryl sulfate et le lauryl éther sulfate, et leurs sels notamment de sodium.

Selon un mode de réalisation préféré de l'invention, le composé susceptible de provoquer une irritation de la peau est choisi parmi les rétinoïdes, les α -hydroxyacides, les β-hydroxyacides, les acides dicarboxyliques saturés et insaturés tels que l'acide octadécène dioique ou Arlatone DIOC DCA vendu par la société Uniqema, les tensioactifs anioniques, cationiques ou amphotères, l'acide n-octanoyl 5-salicylique, les actifs antiperspirants tels que les sels d'aluminium, l'acide (N-2 hydroxyéthylpiperazine-N-2-éthane) sulfonique (HEPES) et l'acide cinnamique.

Le composé susceptible de provoquer une irritation de la peau peut être présent dans la composition selon la présente invention dans une quantité suffisante pour provoquer une réaction d'irritation de la peau. A titre d'exemple, il peut être présent dans une teneur allant de 0,0001 à 70% en poids, de préférence de 0,01 à 50% en poids et mieux de 0,1 à 30% en poids par rapport au poids total de la composition.

Dans les compositions dermocosmétiques selon l'invention, l'extrait de milieu conditionné peut être combiné avec des rétinoïdes ou des corticostéroïdes, ou associé avec des anti-radicaux libres, avec des alpha-hydroxy ou alpha-céto acides ou leurs dérivés, ou encore des bloqueurs de canaux ioniques.
Les compositions dermocosmétiques selon l'invention peuvent, en outre, contenir des additifs inertes ou même pharmacodynamiquement ou cosmétiquement actifs ou des combinaisons des ces additifs et notamment : des agents mouillants, des agents dépigmentant tels que l'hydroquinone, l'acide azelaïque, l'acide caféïque ou l'acide kojique ; des émollients ; des agents hydratants comme le glycérol, le PEG-400, l'urée ; des agents anti-âge, des agents antiséborrhéiques ou antiacnéiques, tels que la S-carboxyméthylcystéine, la S-benzyl-cystéamine, leurs sels et leurs dérivés, ou le peroxyle de benzoyle ; des antibiotiques comme l'érythromycine et ses esters, la néomycine, la clindamycine et ses esters, les tétracyclines ; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chloro 3-methyl 1,2,4-benzothiadiazine 1,1-dioxyde) et le Phénytoïne (5,4-diphényl-imidazoline 2,4-dione) ; des agents anti-inflammatoires non stéroïdiens, des carotenoïdes et notamment le β-carotène ; des agents anti-psoriasiques tels que l'anthraline et ses dérivés et enfin, les eicosa-5,8,11,14-tétraénoïque et eicosa-5,8,11-trynoïque, leurs esters et les amides.

### ACTIFS ADDITIONNELS

Le milieu conditionné ou ses extraits utilisé selon l'invention peut également être associé à au moins 0,00001 % à 95 % en poids d'un agent anti-inflammatoire, un autre agent apaisant ou leur mélange.

Comme exemples « d'agents anti-inflammatoires », on peut citer :
- un antagoniste de cytokines inflammatoires ;
- un anti-inflammatoire stéroïdien (Hydrocortisone, betaméthasone, dexaméthasone etc..) ;
- un anti-inflammatoire non stéroïdien comme l'aspirine;
et leurs mélanges.

Les agents anti-inflammatoires sont de préférence présents dans les compositions conformes à l'invention à une concentration pouvant varier entre 0,00001 et 10 % en poids environ par rapport au poids total de la composition. Encore plus préférentiellement, la concentration en composé anti-inflammatoire peut varier entre 0,0005% et 2 % en poids par rapport au poids total de la composition.

En particulier, l'autre agent apaisant pourra avantageusement être choisi parmi l'allantoine, l'acide bêta-glycyrrhétinique, les extraits en contenant comme par exemple l'extrait de Glycyrrhiza Glabra (réglisse) et les complexes en contenant comme le complexe allantoïne/acide glycyrrhétinique ; les planctons, lyophilisés ou non, leurs extraits et leurs complexes ; les eaux et extraits de fleurs et de plantes : eaux de camomille, de tilleul, rose, extraits de bouleau ; le bisabolol ; les huiles essentielles par exemple de coriandre ; les algues notamment du type *Laminaria* (par exemple rouges ou brunes) tel que l'extrait d'algue brune *Padina Pavonica* comme HPS 3 PADINA PAVONICA commercialisé par la société Alban Muller ; l'acide acexamique et l'acide transexamique (acide trans-4, aminométhylcyclohexane carboxylique) ; l'acide ursolique et les extraits en contenant comme l'extrait de feuille de romarin ; les polysaccharides contenant du fucose, comme le FUCOGEL 1000, vendu par la société Solabia ; les électrolytes et en particulier un mélange aqueux comme le « mélange des sels de la mer Morte » (« Dead Sea Bath Salts ») ; les acides aminés comme les Sepicalm S et VG de Seppic et les sels divalents de magnésium tel que le magnésium gluconate.

Les compositions selon l'invention, en particulier cosmétique ou dermatologique, peuvent notamment être préparée par procédé comprenant une première phase au cours de laquelle on prépare un milieu de culture conditionné, éventuellement un extrait d'un tel milieu de culture conditionné.

Ce procédé comprend au moins les étapes suivantes:
a) culture de cellules dérivées de l'épithélium intestinal sur un support dans un premier milieu nutritif pendant un temps suffisant pour obtenir leur différenciation
b) préparation d'un second milieu de culture cellulaire dans une enceinte tapissée de cellules de sang périphériques ou de leurs dérivées, notamment de leucocytes,
c) récupération de la culture de cellules différenciées sur le support, en particulier un support poreux, obtenue à l'issue de l'étape a) et transfert dans le second milieu de culture obtenu à l'issue de l'étape b)
d) mise en contact de ladite culture de cellules dérivées de l'épithélium intestinal différenciées dans le second milieu de culture, avec une culture de microorganismes comprenant au moins des probiotiques, qui peuvent être par exemple de l'espèce *Lactobacillus,* pendant un temps suffisant pour qu'il y ait une interaction entre les cellules,
e) élimination des cultures cellulaires et récupération du second milieu de culture cellulaire, débarrassé des leucocytes, pour obtenir un milieu conditionné
f) incorporation du milieu conditionné ou d'un extrait de celui-ci dans une composition cosmétique ou dermatologique.

De préférence, le support à l'étape a) est un support poreux. Par support poreux, on entend en particulier un insert dont la base comprend des pores.

Par exemple, la taille des pores pourra varier de 0,001 à 10 µm de préférence supérieure ou égale à 0,3µm. A titre d'exemple non limitatif, la base de l'insert poreux convenant à l'invention, pourra ainsi comprendre une matrice poreuse de collagène, comprenant optionnellement des glycosaminoglycanes et/ou des fibroblastes, un gel ou une membrane d'acide hyaluronique et/ou de collagène et/ou de fibronectine et/ou de fibrine, une membrane semi-perméable de nitrocellulose, de nylon, de téflon, de polycarbonate ou de polyéthylène ou de polypropylène ou de polyethylène térephtalate (PET), une membrane inorganique Anopore^{®} semi-perméable, une membrane d'acétate de cellulose, une membrane semi-perméable Biopore-CM^{®}, une membrane semi-perméable de polyester et une membrane d'acide polyglycolique.

Le temps de contact à l'étape a) sera adapté par l'homme du métier mais sera généralement compris de quelques heures à quelques jours, notamment entre 1 jour et 35 jours, par exemple de 18 à 22 jours, de préférence d'environ 21 jours. Les cellules qui sont disposées initialement en couche unique forment ainsi à l'issue de l'étape a) un système à plusieurs couches de cellules différenciées, tridimensionnel. Avantageusement, le milieu de culture sera renouvelé régulièrement, par exemple tous les 2 jours, pour obtenir une différenciation optimale des cellules dérivées de l'épithélium intestinal, telles que les Caco2.
A l'étape b), les cellules de sang périphérique ou dérivées, en particulier des leucocytes, forment un tapis cellulaire; c'est-à-dire que la surface de l'enceinte est recouverte de façon sensiblement homogène par des leucocytes; ceux-ci peuvent être en monocouche ou en multicouche.
Ces leucocytes seront notamment recueillis à partir de prélèvement sanguin après au moins une étape de séparation, notamment par centrifugation, puis élimination au moins partielle des monocytes. Ils sont ensuite remis en suspension dans un milieu nutritif compatible avec leur viabilité, notamment du milieu RPMI.
De manière générale, les milieux pourront être choisis par l'homme du métier selon ses connaissances générales, et notamment parmi les milieux cités précédemment.

A titre d'exemple d'enceinte convenant à la mise en oeuvre de l'invention, il peut être mentionné des puits de plaques de culture telles que des plaques de culture cellulaire de 6, 12, 24, 48 puits ou de 96 puits, usuellement utilisées en culture de cellules.

Avantageusement, la culture de cellules différenciées sera lavée avec le milieu convenant à la viabilité des leucocytes avant le transfert à l'étape c). Le contact entre les cellules différenciées et les probiotiques, en présence des leucocytes sera effectué pendant un temps permettant l'établissement d'interaction, c'est-à-dire d'échanges chimiques ou biologiques entre les différents types cellulaires.
Au sens de l'invention, on entend désigner par l'expression "échange chimique cellulaire", l'ensemble des signaux figurés par des molécules, libérées à partir d'une cellule, et susceptible d'affecter, à distance, l'activité d'une autre cellule, appartenant ou non au même type cellulaire. Une telle molécule peut être, par exemple et de manière non limitative, un peptide, une protéine, un lipide, un sucre, une hormone stéroïdienne, une catécholamine. Elle peut être libérée sous forme d'une sécrétion.

Il est entendu que cette interaction peut intervenir en l'absence de contact physique direct entre les différents types cellulaires. En particulier, l'agencement dans lequel d'une part les cellules dérivées de l'épithélium intestinal et d'autre part le tapis cellulaire de leucocytes, disposés ou non dans une unique enceinte, sont mis en relation l'un avec l'autre au moyen du milieu de culture dans lequel ils sont incubés, sans que des cellules de la culture de cellules intestinales puissent entrer, directement, en contact avec des cellules d'un autre tapis, par exemple par contact entre les corps cellulaires ou au moyen de prolongements cellulaires.
Avantageusement, les microorganismes probiotiques sont ajoutés au niveau apical sur la culture de cellules intestinales.
Le temps convenant à l'établissement de cette interaction sera de quelques heures à plusieurs jours, généralement d'au moins 6 heures, de préférence d'au moins 10, notamment supérieur ou égal à 16 heures, mais pourra être prolongé sans inconvénient. On laisse par exemple les probiotiques en contact avec les cellules intestinales dans l'enceinte comportant les leucocytes pendant 6 à 36 heures.
Le milieu de culture est ensuite récupéré en le séparant de l'ensemble des cellules, par exemple en le récoltant au niveau basolatéral: ce milieu conditionné a été influencé par les deux types cellulaires et leur interrelation.
Comme indiqué précédemment, le milieu de culture ainsi récupéré, aussi appelé "milieu conditionné", contient de l'IL-10, généralement à une concentration supérieure ou égale à 20 pg/ml de milieu, notamment de 50 à 200 pg/ml.

Il peut ensuite subir des étapes de concentration, extraction, fractionnement connues en soi de l'homme du métier, avant l'introduction à titre d'ingrédient, dans une composition, notamment cosmétique ou pharmaceutique ou dermocosmétique selon l'invention.

L'invention sera illustrée plus en détail dans les exemples qui suivent.

### Exemple 1: Préparation d'un milieu conditionné

Des cellules proche des entérocytes humains, CaCo2 (entre le passage 60 à 65) sont ensemencés à la densité de 2.5 10⁵ cellules/ml dans un insert de culture de 25mm (ayant des nucleopore de 0,4µm, Becton Dickinson, Basel, SUISSE). Ces inserts sont placés dans une boites de culture (Nunc) et cultivés pendant 18 à 22 jours à 37°C/10% CO2 dans du DMEM (contenant de la glutamine et une forte concentration de glucose (Amimed, Allschwill, SUISSE) supplémenté avec des acides aminés non essentiels (Gibco, BRL), 10 mg/ml gentamycine (GIBCO BRL), et 0.1% de penicilline/streptomycine (10 000IU/ml et 10 000UG/ml) (GIBCO). Le milieu de culture est changé tous les 2 jours jusqu'à ce que les cellules soient complètement différentiées (21 jours). Une mesure de la résistance électrique trans épithéliale est déterminée continuellement lorsque les cellules CaCo2 sont confluentes en monocouche en utilisant une électrode Multicell-ERS (voltmètre/ohmmètre).
Par ailleurs des leucocytes sanguins de volontaires sains sont isolées à partir de sang périphérique de donneurs sains non apparentés, par centrifugation sur Lymphoprep. Les suspensions cellulaires (10⁷ cellules/ml) sont déposées dans une boîte de pétri et une incubation de 1h30 à 37°C permet l'adhésion des monocytes. Ainsi, les leucocytes, majoritairement des lymphocytes T, contenus dans la population de cellules non adhérentes sont purifiés en utilisant leur propriété à former des rosettes en présence de globules rouges de mouton. Ces derniers sont ensuite éliminés par un choc osmotique en présence de NH₄Cl (8,7mg/l). Dans tous les cas, la viabilité de la suspension de leucocytes est supérieure à 95%.

Ces leucocytes sont alors dilués dans du RPMI 1640 contenant 20% de sérum humain AB décomplémenté (56°C, 30 minutes, Sigma, St Louis, Missouri, USA).

Le modèle de coculture Caco2/leucocytes peut alors être réalisé. Pour cela les inserts de culture cellulaire de CaCo2 sont lavés 2 fois dans du milieu RPMI 1640 et transférés dans une plaque de culture de 6 puits contenant du milieu RPMI, préalablement tapissés avec des leucocytes (2x10⁶ cellules/ml) fraîchement purifiés. Ainsi, le leucocytes sont au niveau basolatéral et les CaCo2 sont au niveau apical.

La stimulation par des probiotiques de la cocultures CaCo2/leucocytes (globules blancs du sang périphériques) se réalise suivant les condition décrites ci-après:
Ainsi, au niveau apical, sont ajoutés 1x10 ⁷ cfu/ml de probiotiques. Le milieu de culture seul est utilisé comme contrôle négatif. Après la stimulation pendant de 6 à 36h (37°C, 10% CO2), de la coculture, le milieu conditionné est récolté au niveau basolatéral.
On utilise en particulier comme probiotique *Lactobacillus paracasei,* notamment la souche déposée à la CNCM sous le n°CNCMI-2116

### Exemple 2:

Une lignée de cellules intestinales CaCO-2 est mise en culture sur des inserts de 10,5 mm (Becton Dickinson) à raison de 2 x 105 cellules/puits. Ces inserts sont ensuite placés en culture dans une plaque de 12 puits (Nunc). Les cellules sont alors cultivées durant 21 jours à 37°C/10%CO2 dans du DMEM supplémenté avec 10% FCS et 0.1% de penicilline/streptomycine (10'000 UI/mL, Gibco BRL).

Les cellules mono-nucléaires de sang périphérique (leucocytes) humain sont purifiées à partir de poche de sang «buffy coats» par centrifugation à travers une colonne Ficoll-Hypaque 1077 (Pharmacia) puis sont resuspendues dans un milieu RPMI complet supplémenté de sérum humain AB (Gibco BRL)Les leucocytes (2 x 106 cellules/mL) sont alors ajoutées dans le compartiment baso-latérale des cultures « trans-well » lorsque celles-ci présentent une couche confluente de cellules CaCO-2 qui ont été préalablement lavées de leur milieu.

Les co-cultures ainsi établies sont stimulées en ajoutant 1 x 10⁷ CFU/ml de probiotiques au niveau de la surface apicale de la mono-couche de cellules épithéliales (CaCO-2). Le système est en suite incubé durant 16h à 37°C/5% CO2. 150 µg/ml de Gentamicine sont ajoutés au milieu après 4h d'incubation.
En fin d'incubation (16h) le milieu se trouvant dans le compartiment baso-latérale est prélevé pour être testé.

Le tableau suivant illustre le profile de cytokines de milieux conditionnés issus de la stimulation avec 1 x 10⁷ CFU/mL de Lactobacillus paracasei et de Bifidobacterium longum durant 16h.

| | IL10 pg/ml | IFN gamma pg/ml |
|---|---|---|
| B longum | 170 | 110 |
| L. paracasei | 80 | 40 |
| B longum + L. paracasei | 110 | 40 |

Ces résultats montrent que les probiotiques induisent préférentiellement la production de cytokine régulatrice IL-10 plutôt que la cytokine pro-inflammatoire IFN-γ.

### Exemple 3 : Evaluation de l'inhibition de l'inflammation dans un modèle de peau humaine en survie

Du milieu conditionné est préparé selon le protocole de l'exemple 1, en utilisant 1 x 10⁷ CFU/ml de *Lactobacillus paracasei.*
Un modèle de peau humaine maintenue en survie stimulée par un neuromédiateur (la substance P, SP) a été utilisé. Ce neuromédiateur est en effet l'un des agents responsable de la réponse inflammatoire. A côté de ses effets vasculaires (oedème, vasodilatation, expresssion d'ELAM-1 sur la paroi des cellules endothéliales), la SP dans les conditions du test induit également des réactions biochimiques avec libération de médiateurs pro-inflammatoires (IL1α, IL6, TNFα) ainsi qu'une dégranulation des mastocytes (Lembeck F, 1983; Matsuda H et coll., 1989 ; Weidner C et coll., 2000).
L'évaluation a été réalisée histologiquement (évaluation de l'oedème, modifications du diamètre des capillaires et du nombre de mastocytes dégranulés) et biochimiquement (dosage de TNFα).

Des fragments de peau de donneurs différents ont été déposés dans des inserts eux-mêmes disposés en suspension au-dessus de puits de culture. Du milieu (Milieu Essentiel Minimum de Dulbecco, D-MEM) (antibiotiques, SVF) a été ajouté dans le fond des puits, un passage s'effectuant par diffusion lente entre les deux compartiments par l'intermédiaire d'une membrane poreuse (0,45 µm). 5 heures de ré-équilibrage sont nécessaires avant de débuter le protocole.

Au terme des 5 heures de ré-équilibrage, les milieux conditionnés stimulés par L. paracasei (ou non) a été ajouté en prétraitement dans le milieu de culture D-MEM à la concentration de 30%. Les fragments de peau ont alors été maintenus en culture d'organes pendant 24 heures dans une étuve à atmosphère humide, à 37°C et en présence de 5% de CO2.
A J1 le modèle expérimental d'inflammation a été réalisé en ajoutant 10 µM de substance P dans le milieu de culture. Les milieux nutritifs ont été renouvelés et 24 heures d'incubation supplémentaires ont été effectuées pour toutes les conditions.

### Une étude comparative a ainsi été réalisée entre les 8 conditions suivantes:

### Modèle de stimulation par SP en comparaison avec la peau témoin :

- peau témoin (condition de base : peau non stimulée, non traitée) avec milieu D-MEM,
- peau stimulée par la substance P 5 µM en milieu D-MEM,
contrôle absolu (**condition A**):
- peau cultivée avec du milieu nutritif (RPMI) à 30% en milieu D-MEM
- peau stimulée par la substance P et cultivée avec du milieu nutritif (RPMI) à 30% dans le milieu D-MEM
contrôle négatif (**condition B**):
- peau cultivée avec du milieu nutritif (RPMI) à 30% issu d'une coculture CaCO2 / PBMC non stimulée par des ferments lactiques
- peau stimulée par la substance P et cultivée avec le milieu conditionné non stimulée par du *Lactobacillus paracasei*
contrôle positif (**condition C**):
- peau cultivée avec du milieu nutritif (RPMI) à 30% issu d'une coculture CaCO2 / PBMC stimulée par du *Lactobacillus paracasei*
- peau stimulée par la substance P et cultivée avec le milieu conditionné stimulé par L. paracasei

### 1- Evaluation histologique de l'oedème et des modifications du diamètre des capillaires

Les fragments de peaux ont été fixés dans le liquide de Bouin et inclus en paraffine. Après coloration par l'hémalun-éosine, 2 critères ont été évalués au niveau du derme: le calibre des capillaires et l'oedème.
Les résultats sont les suivants

### a) Evaluation du % global de capillaires dilatés

Modèle de stimulation par SP en comparaison avec la peau témoin :
L'application de la SP induit une vasodilatation statistiquement significative par rapport à la peau témoin : 84,5 versus 58,3% (p < 0,05).
Contrôle absolu (**condition A**):
Comme dans le modèle expérimental, une dilatation statistiquement significative des capillaires a été observés après application de la substance P : 78,3 versus 61,5% (p < 0,05).
Contrôle négatif (**condition B**):
Les résultats sont similaires à ceux obtenus avec la condition A, une dilatation statistiquement significative des capillaires est observés après application de la substance P, témoignant de l'absence d'un effet anti-inflammatoire du milieu conditionné non stimulé: 82,2 versus 56,7% (p < 0,05).
Contrôle positif (condition C):
Aucune modification du % de capillaires dilatés entre les conditions C et C+SP (60,9 versus 54,9% ), témoignant de l'effet anti-inflammatoire du milieu conditionné stimulé par *Lactobacillus paracasei.* Ce résultat est confirmé par l'existence d'une différence statistiquement significative entre la condition C + SP et les autres conditions : témoin + SP, A + SP et B + SP (p < 0,05).

### b) Mesure de la surface moyenne des capillaires

Les résultats concernant la mesure de la surface moyenne des capillaires sont similaires à ceux obtenus avec l'analyse du % global de capillaires dilatés.

Modèle de stimulation par SP en comparaison peau témoin :
L'application de la SP induit une vasodilatation statistiquement significative par rapport à la peau témoin : 164,5 µm² versus 69 µm² (p < 0,05).
Contrôle absolu **(condition A):**
Comme dans le modèle expérimental, nous observons une dilatation statistiquement significative des capillaires après application de la substance P : 127,8 µm² versus 57 µm² (p < 0,05).
Contrôle négatif (**condition B**):
Les résultats sont similaires à ceux obtenus avec la condition A, une dilatation statistiquement significative des capillaires est observés après application de la substance P, témoignant de l'absence d'un effet anti-inflammatoire du milieu conditionné non stimulé : 154 µm² versus 67 µm² (p < 0,05).
Contrôle positif (**condition C**):
Une diminution significative de la surface des capillaires dilatés pour la condition C+SP par rapport à la condition C (59,2 µm² versus 87µm²), témoignant de l'effet anti-inflammatoire du milieu conditionné stimulé par L. paracasei (p < 0,05). Ce résultat est confirmé par l'existence d'une différence statistiquement significative entre la condition C + SP et les autres conditions : témoin + SP, A + SP et B + SP (p < 0,05).

### Evaluation de l'oedème dermique

Modèle de stimulation par SP en comparaison peau témoin :
L'application de la SP induit un oedème statistiquement significatif par rapport à la peau témoin : score de 1,8 versus 0,7 (p < 0,05).

### Contrôle absolu (condition A):

Comme dans le modèle expérimental, nous observons un oedème statistiquement significatif après application de la substance P : score de 1,9 versus 1,3 (p < 0,05).

### Contrôle négatif (condition B):

Les résultats sont similaires à ceux obtenus avec la condition A : un oedème a été noté après application de la substance P, témoignant de l'absence d'un effet anti-inflammatoire du milieu conditionné non stimulé: score de 1,9 versus 0,8 (p < 0,05).

### Contrôle positif (condition C):

Aucune augmentation de l'oedème dans la condition C+ SP par rapport à la condition C (0,9 versus 1) n'a été obtenu, témoignant de l'effet anti-inflammatoire du milieu conditionné stimulé par L. paracasei. Ce résultat est confirmé par l'existence d'une différence statistiquement significative entre la condition C + SP et les autres conditions : témoin + SP, A + SP et B + SP (p < 0,05).

### 2- Evaluation histologique de la dégranulation des mastocytes

Les mastocytes présents dans le derme ont été révélés en bleu-violet par la coloration au bleu de toluidine. Histologiquement, un aspect bleu-violet plus ou moins intense et granuleux des mastocytes en rapport avec la présence plus ou moins importante dans leur cytoplasme de granulations basophiles et métachromatiques contenant notamment de l'histamine a été observé.

Dans le modèle de peau maintenue en survie stimulé par la SP, nous observons une diminution statistiquement significative du % de mastocytes fortement granulés (score 3) a été observés: 32,1% versus 53% au niveau des peaux témoins et une augmentation du % de cellules de score 1 : 26,5 versus 9,5 %. Nous obtenons donc bien une dégranulation induite par la substance P.

### Contrôle absolu (condition A):

Comme dans le modèle expérimental, nous observons une diminution du % de mastocytes de score 3 : 14,8 versus 41,7 % et une augmentation du score 1 : 36,9 versus 12,2% (p < 0,05).

### Contrôle négatif (condition B):

Les résultats sont similaires à ceux obtenus avec la condition A avec diminution du % de mastocytes de score 3 : 17,5 versus 38,5% et une augmentation du % de cellules de score 1 :33,3 versus 20,4%: (p < 0,05). Ces résultats permettent de conclure que le milieu conditionné non stimulé ne protège pas vis-à-vis de la dégranulation induite par la substance P

### Contrôle positif (condition C):

Aucune différence significative entre le % de mastocytes de score 3 n'a été obtenu dans la condition C et celui observé dans la condition C+SP (score de 46,9 versus 37,2). Cette absence de différence peut donc témoigner d'un effet protecteur modéré du milieu conditionné stimulé par L. paracasei vis à vis de la dégranulation induite par la substance P. Cet effet est partiel car le % de mastocytes de score 1 est augmenté mais de niveau plus faible que dans les autres conditions : témoin + SP, A + SP et B + SP (p < 0,05). Néanmoins, cette augmentation étant faible, nous retrouvons une différence statistiquement significative entre la condition C + SP et les autres conditions : témoin + SP, A + SP et B + SP (p < 0,05).

### 3- Dosage de cytokine pro-inflammaoire

La modulation de la sécrétion de cytokines telle que le TNF-alpha a été recherchée.
Le dosage de cette cytokine a été réalisé par une technique d'immuno-essai avec lecture spectrophotométrique de la concentration (pg/ml) (kits de dosage Chemicon International, INC). Les fragments de peau ayant la même surface (vérification du poids de chaque fragment), le dosage a été réalisé à partir des surnageants de culture.

Les résultats du dosage de TNF sont les suivants
Dans la peau témoin et la condition A correspondant au contrôle absolu, l'application de la SP induit une augmentation statistiquement significative du taux de TNF par rapport à la peau témoin : respectivement score de 31,5 versus 10,7 et score de 28,6 versus 8,32 pg/ml (p < 0,05).
La condition C+SP est statistiquement différente de la condition peau témoin +SP (p=0,017) suggérant que le milieu conditionné L. paracasei, diminue la production de TNF-α (taux de 10,9 versus 16,3 pg/ml).

### Conclusion

Dans ce modèle de peau humaine maintenue en survie, un effet anti-inflammatoire du milieu conditionné stimulé par L. paracasei (C), a été mis en évidence avec diminution importante et statistiquement significative de la vasodilatation, de l'oedème induit, de la dégranulation des mastocytes et de la libération de TNF induits par la substance P par rapport aux conditions A + SP (milieu RPMI) et B + SP (milieu conditionné non stimulé).

### Exemple 4 : Evaluation de l'inhibition de l'inflammation dans un modèle de peau humaine en survie

Du milieu conditionné est préparé selon le protocole de l'exemple 1, en utilisant 1 x 10⁷ CFU/ml de *Lactobacillus paracasei* ou *Bifidobacterim longum*
Un modèle de peau humaine maintenue en survie stimulée par un neuromédiateur (la substance P, SP) a été utilisé. Ce neuromédiateur est en effet l'un des agents responsable de la réponse inflammatoire et des effets vasculaires (oedème, vasodilatation, expresssion d'ELAM-1 sur la paroi des cellules endothéliales), L'évaluation a été réalisée histologiquement (évaluation de l'oedème, modifications du diamètre des capillaires)

Des fragments de peau de donneurs différents ont été déposés dans des inserts eux-mêmes disposés en suspension au-dessus de puits de culture. Du milieu (Milieu Essentiel Minimum de Dulbecco, D-MEM) (antibiotiques, SVF) a été ajouté dans le fond des puits, un passage s'effectuant par diffusion lente entre les deux compartiments par l'intermédiaire d'une membrane poreuse (0,45 µm). 5 heures de ré-équilibrage sont nécessaires avant de débuter le protocole.

Au terme des 5 heures de ré-équilibrage, le milieu conditionnés stimulé (ou non) par L. paracasei et/ou B. longum a été ajouté en prétraitement dans le milieu de culture D-MEM à la concentration de 30%. Les fragments de peau ont alors été maintenus en culture d'organes pendant 24 heures dans une étuve à atmosphère humide, à 37°C et en présence de 5% de CO2.
A J1 le modèle expérimental d'inflammation a été réalisé en ajoutant 10 µM de substance P dans le milieu de culture. Les milieux nutritifs ont été renouvelés et 24 heures d'incubation supplémentaires ont été effectuées pour toutes les conditions.

### Une étude comparative a ainsi été réalisée entre les 8 conditions suivantes:

### Modèle de stimulation par SP en comparaison avec la peau témoin :

- peau témoin (condition de base : peau non stimulée, non traitée) avec milieu D-MEM,
- peau stimulée par la substance P 5 µM en milieu D-MEM,

### contrôle absolu (condition 1):

- peau cultivée avec du milieu nutritif RPMI à 30% en milieu D-MEM
- peau stimulée par la substance P et cultivée avec du milieu nutritif (RPMI) à 30% dans le milieu D-MEM

### contrôle négatif (condition 2):

- peau cultivée avec du milieu nutritif (RPMI) à 30% issu d'une coculture CaCO2 / PBMC non stimulée par des ferments lactiques
- peau stimulée par la substance P et cultivée avec le milieu conditionné non stimulée par des probiotiques

### condition 3:

- peau cultivée avec du milieu nutritif (RPMI) à 30% issu d'une coculture CaCO2 / PBMC stimulée par du *Lactobacillus paracasei+ Bifidobacterium Longum*
- peau stimulée par la substance P et cultivée avec le milieu conditionné stimulé par *L. paracasei* + *B. Longum*

### condition 4:

- peau cultivée avec du milieu nutritif (RPMI) à 30% issu d'une coculture CaCO2 / PBMC stimulée par du *Lactobacillus paracasei*
- peau stimulée par la substance P et cultivée avec le milieu conditionné stimulé par *L. paracasei*

### condition 5:

- peau cultivée avec du milieu nutritif (RPMI) à 30% issu d'une coculture CaCO2 / PBMC stimulée par du *Bifidobacterium Longum*
- peau stimulée par la substance P et cultivée avec le milieu conditionné stimulé par *B. Longum*

### Evaluation histologique de l'oedème et des modifications du diamètre des capillaires

Les fragments de peaux ont été fixés dans le liquide de Bouin et inclus en paraffine. Après coloration par l'hémalun-éosine, 2 critères ont été évalués au niveau du derme: le calibre des capillaires et l'oedème.
Les résultats sont les suivants

### a) Evaluation du % global de capillaires dilatés

### Contrôle absolu (condition 1):

Comme dans le modèle expérimental, une dilatation statistiquement significative des capillaires a été observés après application de la substance P : 86,6 versus 63,8% (p < 0,05).

### Contrôle négatif (condition 2):

Les résultats sont similaires à ceux obtenus avec la condition 1, une dilatation statistiquement significative des capillaires est observés après application de la substance P, témoignant de l'absence d'un effet anti-inflammatoire du milieu conditionné non stimulé: 86,1 versus 75,3% (p < 0,05).

### Condition 3:

Aucune modification du % de capillaires dilatés entre les conditions 3 et 3+SP (72,4 versus 72,4%), témoignant de l'effet anti-inflammatoire du milieu conditionné stimulé par *Lactobacillus paracasei* + *B. Longum.* Ce résultat est confirmé par l'existence d'une différence statistiquement significative entre la condition 3 + SP et les autres conditions : témoin + SP, 1 + SP et 2 + SP (p < 0,05).

### Condition 4:

Aucune modification du % de capillaires dilatés entre les conditions 4 et 4+SP (73,2 versus 71,6%), témoignant de l'effet anti-inflammatoire du milieu conditionné stimulé par *Lactobacillus paracasei.* Ce résultat est confirmé par l'existence d'une différence statistiquement significative entre la condition 4 + SP et les autres conditions : témoin + SP, 1 + SP et 2 + SP (p < 0,05).

### Condition 5:

Aucune modification du % de capillaires dilatés entre les conditions 5 et 5+SP (75,6 versus 66,9%), témoignant de l'effet anti-inflammatoire du milieu conditionné stimulé par B. Longum. Ce résultat est confirmé par l'existence d'une différence statistiquement significative entre la condition 5+ SP et les autres conditions : témoin + SP, 1 + SP et 2 + SP (p < 0,05).

### b) Mesure de la surface moyenne des capillaires

Les résultats concernant la mesure de la surface moyenne des capillaires sont similaires à ceux obtenus avec l'analyse du % global de capillaires dilatés.

### Contrôle absolu (condition 1):

Comme dans le modèle expérimental, nous observons une dilatation statistiquement significative des capillaires après application de la substance P : 172,6 µm² versus 95,36 µm² (p < 0,05).

### Contrôle négatif (condition 2):

Les résultats sont similaires à ceux obtenus avec la condition 1, une dilatation statistiquement significative des capillaires est observés après application de la substance P, témoignant de l'absence d'un effet anti-inflammatoire du milieu conditionné non stimulé : 163,8 µm² versus 110,3 µm² (p < 0,05).

### Condition 3:

Une diminution significative de la surface des capillaires dilatés pour la condition 3+SP par rapport à la condition 3 (121,9µm² versus 102µm²), témoignant de l'effet anti-inflammatoire du milieu conditionné stimulé par L. paracasei+B. Longum (p < 0,05). Ce résultat est confirmé par l'existence d'une différence statistiquement significative entre la condition 3 + SP et les autres conditions : 1 + SP et 2 + SP (p < 0,05).

### Condition 4:

Une diminution significative de la surface des capillaires dilatés pour la condition 3+SP par rapport à la condition 4 (112,3µm² versus 117,4µm²), témoignant de l'effet anti-inflammatoire du milieu conditionné stimulé par L. paracasei (p < 0,05). Ce résultat est confirmé par l'existence d'une différence statistiquement significative entre la condition 4 + SP et les autres conditions :1 + SP et 2 + SP (p < 0,05).

### Condition 5:

Une diminution significative de la surface des capillaires dilatés pour la condition 5+SP par rapport à la condition 5 (110,4µm² versus 105,7µM²), témoignant de l'effet anti-inflammatoire du milieu conditionné stimulé par B. Longum (p < 0,05). Ce résultat est confirmé par l'existence d'une différence statistiquement significative entre la condition 5 + SP et les autres conditions : 1 + SP et 2 + SP (p < 0,05).

### Evaluation de l'oedème dermique

### Contrôle absolu (condition 1):

Comme dans le modèle expérimental, nous observons un oedème statistiquement significatif après application de la substance P : score de 1,7 versus 1,05 (p < 0,05).

### Contrôle négatif (condition 2):

Les résultats sont similaires à ceux obtenus avec la condition 1: un oedème a été noté après application de la substance P, témoignant de l'absence d'un effet anti-inflammatoire du milieu conditionné non stimulé: score de 1,4 versus 1,3 (p < 0,05).

### Condition 3:

Aucune augmentation de l'oedème dans la condition 3+ SP par rapport à la condition 3 (1,23 versus 1,17) n'a été obtenu, témoignant de l'effet anti-inflammatoire du milieu conditionné stimulé par *L. paracasei+ B.Longum.* Ce résultat est confirmé par l'existence d'une différence statistiquement significative entre la condition 3 + SP et les autres conditions : 1 + SP et 2 + SP (p < 0,05).

### Condition 4:

Aucune augmentation de l'oedème dans la condition 4+ SP par rapport à la condition 4 (1,37 versus 1,38) n'a été obtenu, témoignant de l'effet anti-inflammatoire du milieu conditionné stimulé par *L. paracasei.* Ce résultat est confirmé par l'existence d'une différence statistiquement significative entre la condition 3 + SP et les autres conditions : 1 + SP et 2 + SP (p < 0,05).

### Condition 5:

Aucune augmentation de l'oedème dans la condition 5+ SP par rapport à la condition 5 (1,4 versus 1,06) n'a été obtenu, témoignant de l'effet anti-inflammatoire du milieu conditionné stimulé par *B. Longum.* Ce résultat est confirmé par l'existence d'une différence statistiquement significative entre la condition 3 + SP et les autres conditions : 1 + SP et 2 + SP (p < 0,05).

### Exemple 5: Compositions

| **Composition pour préparer la peau au soleil** | |
|---|---|
| Milieu conditionné stimulé* | 2,5% |
| Conservateurs | 1,35% |
| Sodium citrate | 0,035% |
| PEG-40 | 1,25% |
| Pentaerythrityl tetraethylhexanoate | 4% |
| Glycerin | 7% |
| Sorbitan tristearate | 0,3% |
| Prunus armeniaca Kermel oil | 2% |
| Cetyl alcohol | 0,7% |
| Propylene glycol | 2% |
| Triethanolamine | 0,4% |
| Cyclohexasiloxane | 2% |
| Carbomer | 0,75% |
| Tocopherol | 1% |
| Silica | 2% |
| Ascorbyl glucoside | 0,1% |
| Polycaprolactone -béta carotène | 5% |
| Eau qsp | 100% |

| **Composition contenant des filtres solaires** | |
|---|---|
| Milieu conditionné stimulé** | 3,5% |
| Mélange d'alcool cetylstearylique et d'alcool cetylstearylique oxyéthylene (33OE) 80/20 | 7,0% |
| Mélange de mono et de distearate de glycerol | 2,0% |
| Alcool cetylique | 1,5% |
| Poly dimethylsiloxane | 1,5% |
| Huile de vaseline | 15,0% |
| Butyl methoxydibenzoylmethane | 3,0% |
| Octocrylene | 7,0% |
| Glycerine | 20,0% |
| Eau déminéralisée | qsp 100% |

| **Crème** | |
|---|---|
| Extrait de Milieu conditionné* | 1,5% |
| Glyceryl stearate et PEG 100 stéarate | 5,0% |
| Isohexadecane | 8,0% |
| Beurre de Karité | 5,0% |
| Glycérine | 3,0% |
| Carbopol 981 0,2% | 0,2% |
| Lubragel | 5,0% |
| Phenoxyéthanol | 1,0% |
| Acide citrique | 1,0% |
| BHT | 0,05% |
| Eau | qsp 100% |

| | |
|---|---|
| *obtenu selon l'exemple 1 **obtenu selon l'exemple 1, mais avec un mélange de L.paracasei et de B. longum *obtenu selon l'exemple 1 et lyophilisé | |

## Revendications

1. Milieu de culture cellulaire conditionné ou un extrait de celui-ci, l'extrait étant obtenu par concentration et/ou lyophilisation, ou composition le contenant, pour son utilisation pour le traitement des signes de l'inflammation et/ou des désordres immunitaires, ledit milieu étant susceptible d'être obtenu par contact avec au moins une culture de cellules du tractus digestif, au moins des microorganismes probiotiques et en outre des cellules de sang périphérique.

2. Milieu de culture, extrait ou composition pour son utilisation selon la revendication 1, **caractérisé en ce que** les cellules de sang périphériques sont des leucocytes.

3. Milieu de culture, extrait ou composition pour son utilisation selon l'une quelconque des revendications précédentes,**caractérisé en ce que** les cellules digestives sont des cellules épithéliales de l'intestin.

4. Milieu de culture, extrait ou composition pour son utilisation selon l'une quelconque des revendications précédentes **caractérisé en ce qu'**au moins un microorganisme probiotique est choisi dans le groupe suivant: *Saccharomyces, Yarrowia, Kluyveromyces, Torulaspora, Schizosaccharomyces pombe, Debaromyces, Candida, Pichia, Aspergillus, Penicillium, Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus* et *Lactobacillus.*

5. Milieu de culture, extrait ou composition pour son utilisation selon l'une quelconque des revendications précédentes, **caractérisé en ce qu'**au moins un microorganisme probiotique est choisi parmi les bactéries lactiques, les bifidobactéries et les levures Saccharomyces.

6. Milieu de culture, extrait ou composition pour son utilisation selon l'une quelconque des revendications précédentes **caractérisé en ce qu'**au moins un microorganisme est choisi parmi les espèces *Lactobacillus et Bifidobacterium.*

7. Milieu de culture, extrait ou composition pour son utilisation selon l'une des revendications précédentes, pour le traitement des signes de l'irritation de la peau et/ou du cuir chevelu.

8. Milieu de culture, extrait ou composition pour son utilisation selon l'une quelconque des revendications précédentes, pour protéger les cellules cutanées des dégâts causés par les rayonnements UV.

9. Milieu de culture, extrait ou composition pour son utilisation selon l'une des revendications précédentes, pour protéger les cellules des dégâts de leur ADN.

10. Composition pour son utilisation selon l'une quelconque des revendications précédentes,**caractérisé en ce que** la composition est une composition pharmaceutique.

11. Composition selon la revendication précédente, adaptée pour une application topique sur la peau ou les phanères.

12. Composition cosmétique ou dermatologique **caractérisée en ce qu'**elle contient au moins un milieu conditionné ou un extrait de milieu conditionné tel que défini dans l'une des revendications 1 à 6 et au moins un agent ayant un effet secondaire irritant.

## Patentansprüche

1. Konditioniertes Zellkulturmedium oder Extrakt davon, wobei der Extrakt durch Einengen und/oder Lyophilisieren erhalten wird, oder Zusammensetzung, die dieses/diesen enthält, für die Verwendung zur Behandlung von Anzeichen von Entzündung und/oder Immunstörungen, wobei das Medium durch Kontakt mit mindestens einer Kultur von Zellen des Verdauungstrakts, mindestens probiotischen Mikroorganismen und ferner Zellen des peripheren Bluts erhältlich ist.

2. Kulturmedium, Extrakt oder Zusammensetzung für die Verwendung nach Anspruch 1, **dadurch gekennzeichnet, dass** die Zellen des peripheren Bluts Leukozyten sind.

3. Kulturmedium, Extrakt oder Zusammensetzung für die Verwendung nach einem der vorhergehenden Ansprüche, **dadurch gekennzeichnet, dass** die Verdauungszellen Dünndarmepithelzellen sind.

4. Kulturmedium, Extrakt oder Zusammensetzung für die Verwendung nach einem der vorhergehenden Ansprüche, **dadurch gekennzeichnet, dass** mindestens ein probiotischer Mikroorganismus aus der folgenden Gruppe ausgewählt ist: *Saccharomyces, Yarrowia, Kluyveromyces, Torulaspora, Schizosaccharomyces pombe, Debaromyces, Candida, Pichia, Aspergillus, Penicillium, Bifidobacterium, Bacteroides, Fusobacterium, Melissococcus, Propionibacterium, Enterococcus, Lactococcus, Staphylococcus, Peptostrepococcus, Bacillus*, *Pediococcus, Micrococcus, Leuconostoc, Weissella, Aerococcus, Oenococcus* und *Lactobacillus*.

5. Kulturmedium, Extrakt oder Zusammensetzung für die Verwendung nach einem der vorhergehenden Ansprüche, **dadurch gekennzeichnet, dass** mindestens ein probiotischer Mikroorganismus aus Milchsäurebakterien, Bifidobakterien und Saccharomyces-Hefen ausgewählt ist.

6. Kulturmedium, Extrakt oder Zusammensetzung für die Verwendung nach einem der vorhergehenden Ansprüche, **dadurch gekennzeichnet, dass** mindestens ein probiotischer Mikroorganismus aus den Spezies *Lactobacillus* und *Bifidobacterium* ausgewählt ist.

7. Kulturmedium, Extrakt oder Zusammensetzung für die Verwendung nach einem der vorhergehenden Ansprüche zur Behandlung von Anzeichen einer Reizung der Haut und/oder der Kopfhaut.

8. Kulturmedium, Extrakt oder Zusammensetzung für die Verwendung nach einem der vorhergehenden Ansprüche zum Schutz der Hautzellen vor durch UV-Strahlen verursachten Schäden.

9. Kulturmedium, Extrakt oder Zusammensetzung für die Verwendung nach einem der vorhergehenden Ansprüche zum Schutz der Zellen vor Schäden an ihrer DNA.

10. Zusammensetzung für die Verwendung nach einem der vorhergehenden Ansprüche, **dadurch gekennzeichnet, dass** es sich bei der Zusammensetzung um eine pharmazeutische Zusammensetzung handelt.

11. Zusammensetzung nach dem vorhergehenden Anspruch, die für eine topische Anwendung auf die Haut oder die Hautanhangsgebilde ausgelegt ist.

12. Kosmetische oder dermatologische Zusammensetzung, **dadurch gekennzeichnet, dass** sie mindestens ein konditioniertes Medium oder einen Extrakt des konditionierten Mediums nach einem der Ansprüche 1 bis 6 und mindestens ein Mittel mit einer reizenden Nebenwirkung enthält.

## Claims

1. Conditioned cell culture medium or an extract thereof, the extract being obtained by concentration and/or lyophilization, or composition containing same, for use thereof in the treatment of the signs of inflammation and/or of immune disorders, said medium being capable of being obtained by contact with at least one culture of digestive tract cells, at least probiotic microorganisms and, in addition, peripheral blood cells.

2. Culture medium, extract or composition for use thereof according to Claim 1, **characterized in that** the peripheral blood cells are leukocytes.

3. Culture medium, extract or composition for use thereof according to any one of the preceding claims, **characterized in that** the digestive cells are epithelial cells of the intestine.

4. Culture medium, extract or composition for use thereof according to any one of the preceding claims, **characterized in that** at least one probiotic microorganism is chosen from the following group: *Saccharomyces, Yarrowia, Kluyveromyces, Torulaspora, Schizosaccharomyces pombe, Debaromyces, Candida, Pichia, Aspergillus, Penicillium, Bifidobacterium, Bacteroides, Fusobacterium, Melissococcus, Propionibacterium, Enterococcus, Lactococcus, Staphylococcus*, *Peptostrepococcus, Bacillus, Pediococcus, Micrococcus, Leuconostoc, Weissella, Aerococcus, Oenococcus* and *Lactobacillus.*

5. Culture medium, extract or composition for use thereof according to any one of the preceding claims, **characterized in that** at least one probiotic microorganism is chosen from lactic acid bacteria, bifidobacteria and Saccharomyces yeasts.

6. Culture medium, extract or composition for use thereof according to any one of the preceding claims, **characterized in that** at least one microorganism is chosen from the *Lactobacillus* and *Bifidobacterium* species.

7. Culture medium, extract or composition for use thereof according to one of the preceding claims, in the treatment of the signs of irritation of the skin and/or of the scalp.

8. Culture medium, extract or composition for use thereof according to any one of the preceding claims, for protecting skin cells against damage caused by UV radiation.

9. Culture medium, extract or composition for use thereof according to one of the preceding claims, for protecting cells against damage to their DNA.

10. Composition for use thereof according to any one of the preceding claims, **characterized in that** the composition is a pharmaceutical composition.

11. Composition according to the preceding claim, suitable for topical application to the skin or the skin appendages.

12. Cosmetic or dermatological composition, **characterized in that** it contains at least one conditioned medium or one conditioned medium extract as defined in one of Claims 1 to 6 and at least one agent which has an irritant side effect.
